# NEW EUROPEAN PATENT SPECIFICATION

(11) **EP 1 250 914 B2**
(45) Date of publication and mention of the opposition decision: **25.11.2015**
(45) Mention of the grant of the patent: 31.05.2006
(21) Application number: 01109352.3
(22) Date of filing: 17.04.2001
(51) Int. Cl.: A61L 15/20

(54) **Cooling compositions**
Erfrischende Zusammensetzugen
Compositions de refroidissement

(43) Date of publication of application: 23.10.2002
(73) Proprietor: THE PROCTER & GAMBLE COMPANY, Cincinnati, OH 45202 (US)
(72) Inventor: Carlucci, Giovanni, 66100 Chieti (IT); Pesce, Antonella, 65120 Pescara (IT); Meo, Daniela, 84133 Salerno (IT); Di Cintio, Achille, 65126 Pescara (IT)
(74) Representative: Briatore, Andrea

(56) References cited:
- EP-A- 0 997 144
- WO-A1-00/42983
- WO-A1-93/23005
- DE-A1- 2 608 226
- GB-A- 1 095 505
- JP-A- H04 337 395
- US-A- 4 844 883
- US-A- 6 001 341
- DATABASE WPI Derwent Publications Ltd., London, GB; AN 1993-011695 XP002181781 & JP 04 337395 A (KANEBO), 25 November 1992 (1992-11-25)
- PATENT ABSTRACTS OF JAPAN vol. 13, no. 262 (C-608), 16 June 1989 (1989-06-16) & JP 01 066107 A (KANEBO), 13 March 1989 (1989-03-13)
- PATENT ABSTRACTS OF JAPAN vol. 2000, no. 12, 3 January 2001 (2001-01-03) & JP 2000 247859 A (KAO), 12 September 2000 (2000-09-12)
- DATABASE CA [Online] CHEMICAL ABSTRACTS SERVICE, COLUMBUS, OHIO, US; Database accession no. 2000:266234 XP002181779 & JP 2000 119132 A (TOYO AEROSOL INDUSTRY) 25 April 2000 (2000-04-25)
- DATABASE WPI Week 198237 Derwent Publications Ltd., London, GB; AN 1982-77553e XP002181782 & JP 57 126416 A (SEKISUI CHEM IND), 6 August 1982 (1982-08-06)
- DATABASE CA [Online] CHEMICAL ABSTRACTS SERVICE, COLUMBUS, OHIO, US; Database accession no. 2000:214827 XP002181780 & JP 2000 095679 A (NAGOYA SPORTS) 4 April 2000 (2000-04-04)

## Description

### Field of the Invention

The present invention relates to compositions comprising a cooling agent together with an ester derivative as per formulae described herein after. Such compositions are able to provide long lasting cooling effect when applied to the skin and/or mucosal membranes of a mammal, preferably human, while maintaining or even improving skin health and/or mucosal surface health.

### Background of the Invention

Compositions of various types have incorporated within them components which provide cooling sensation to mucosal membranes and/or to skin. Such compositions include toothpastes, mouthwashes, perfumes, lotions, shaving creams, post shaving preparations, shampoos, antiperspirants, deodorants, and pharmaceutical products among many others.

An example of such compositions is the cosmetic composition disclosed in Japanese abstract JP - A-01066107 (Kanebo), 13 March 1989. This composition comprises triethyl citrate as a compatibilizing agent, liquid fatty acid ester having branched chains, ethyl alcohol and water. Such a composition has excellent preservation stability and compatibility with the skin and is able to impart good humectant properties, softness and moist touch to the skin.

It is well established that the "cooling" effect of menthol is a physiological effect due to the direct action of menthol on the nerve endings of the human body responsive for the detection of hot or cold and is not due to latent heat of evaporation/sublimation. It is believed that the menthol acts as a direct stimulus on the cold receptors at the nerve endings which in turn stimulate the central nervous system.

Although menthol is well established as a physiological cooling agent its use alone, in some compositions, is limited by its strong mint odor and its relative irritant aspect on skin.

Several other compounds have been reported in the technical literature as having an odor or flavor similar to menthol and from time to time have been proposed as flavorants or odorants in a variety of topical and ingestible compositions. For example in French Pat. Spec. No. 1,572,332 N,N-Dimethyl 2-ethylbutanamide is reported as having a mint odor and refreshing effect, and the mint odor of N,N-diethyl 2,2-dimethylpropanamide is referred to. A similar effect is reported for N,N-diethyl 2-ethylbutanamide in Berichte 39, 1223, (1906). A mint odor has also been reported for 2,4,6-trimethylheptan-4-ol and 2,4,6-trimethyl help-2-en-4-ol in Parfums-Cosmetiques-Savons, May 1956, pp. 17-20. The cooling effect of menthol and other related terpene alcohols and their derivatives (1970), pp. 39-43. 2,3-p-menthane diol has also been reported as having a sharp cooling taste (Bellstein, Handbuch der Organischen Chemie, 4th Ed. (1923) Vol. 6, p. 744).

There have been also significant efforts to provide cooling effect without the strong mint odor typically associated with menthol.

Cyclohexanol derivatives have also been disclosed for cooling effect and use in various applications, see for example US 5 756 857. Ketal compounds have been disclosed for their cooling effect in US for example US 5 266 592 and US 5 451 404. Carboxamides have also been disclosed for use in a variety of compositions. Two patents describing such materials and compositions are U.S. Pat. No. 4,136,163, Jan. 23, 1979 to Watson et al. and U.S. Pat. No. 4,230,688, Oct. 28, 1980 to Rowsell et al.

Although there have been these significant efforts to provide enhanced cooling properties to a wide variety of products there is still the need to provide further improved performance.

It is therefore an object of the present invention to provide improved cooling compositions, able to deliver long lasting cooling sensation without compromising on the safety profile towards the skin and/or mucosal membrane or even improving it. More particularly, it is a further object of the present invention to deliver such benefits with improved odour profile too.

It has now been found that these objects are achieved by providing a composition comprising a cooling agent together with an ester derivative described herein after. Cooling agents for use herein are typically able to convey a freshness sensation to the user to which the composition is topically applied to without the need to modify the temperature of the external topical surface to which the composition is applied. Particularly suitable cooling agents for use herein are typically those able to stimulate thermo-receptors of skin and/or mucosal surfaces to convey freshness sensation and are selected from the group consisting of borneol, tea tree oil, ketals, carboxamides, cyclohexanol derivatives as described in claim 1 cyclohexyl derivatives as described in claim 1 and mixtures thereof.

Without to be bound by any theory, the preferred cooling agents for use herein have the ability to cause a subjective sensation of freshness. This sensation of freshness is attributed to the stimulation of thermo-receptors of mammal body. Indeed, it is believed that such cooling agents act as a direct stimulus on the cold receptors at the sensory nerve endings, which in turn stimulate the central nervous system. In other words, such cooling agents suitable for use herein are agents able to chemically stimulate body thermo-receptors, thereby providing a freshness/cooling sensation, this without modifying body surface temperature. It is further noticeable that due to the persistence of the stimuli a long lasting cooling perception is delivered, this even after stopping direct contact between the skin and/or mucosal surface and the composition. Advantageously, these freshness properties are delivered without that any wet feeling is perceived on the contact of the composition with skin and/or mucosal membranes.

Advantageously the presence of an ester derivative as per formulae described herein after in the composition herein results in optimized freshness sensation. Indeed, the presence of such an ester derivative on top of the cooling agent provides both faster freshness sensation as well as sustained freshness sensation. Without to be bound by any theory, it is speculated that the ester derivatives as described herein after - for example those of formulae (II) like triethyl citrate - act as a carrier for the cooling agent, to help it migrate from the composition applied on the skin and/or mucosal surface into the outer layers of the skin (stratum corneum) and bring it into direct and prolonged contact with the thermo-receptors of mammal skin and/or mucosal surface. In other words, the ester derivatives used herein help the cooling agents to reach their target, namely thermo-receptors, in faster condition, thereby delivering a more immediate freshness sensation, and maintain the cooling agents in prolonged contact with the thermo-receptors, thereby promoting further long lasting freshness sensation. Actually, the presence of the ester derivatives described herein after, allows a controlled diffusion of the cooling agent through the stratum corneum of the skin, thereby resulting in a sustained freshness sensation even upon prolonged periods of time. Advantageously, the presence of the ester derivatives delivers an optimized freshness sensation while maintaining or even improving skin and/or mucosal membrane health and deodorizising the skin and/or mucosal surface. Indeed, the presence of ester derivatives selected herein not only moisturizes/hydrates and softens the skin, thereby reducing roughness, cracking and skin irritation that would otherwise be typically associated with cooling agents like menthol when used alone, but also deodorizes skin/mucosal surface due to their antimicrobial activity, namely their enzyme inhibitor activity (this later benefit is particularly noticeable upon occurrence of body fluids/exudates). Without to be bound by theory it is speculated that malodor control benefit is due to a double mechanisms: competitive inhibition with the natural substrates on the active side of the enzymes and enzymes inhibition due to acidic pH created by ester hydrolysis of the ester derivatives described herein.

More particularly the present compositions find a particular application to prevent or reduce skin irritation, namely skin rash caused by dermatitis. The main factor which influences the development of skin irritation or skin rash is the contact of the skin with the body fluids, directly or for example contained in hygienic absorbent articles, like diapers or feminine protection absorbent articles. Especially when the water content is high, skin irritation including skin rash can occur easily. Combining the above mentioned compounds results in compositions being particularly suitable in improving comfort, by delivering immediate and long lasting freshness sensation and maintaining or even improving skin health, by reducing skin irritation problem, this while reducing occurrence of malodor emanating from body fluids.

Advantageously all above-mentioned benefits are obtained by using only two classes of actives, thereby resulting in compositions having a better cost/performance ratio.

Yet a further advantage of the presence of an ester derivative as described herein in the composition herein, is its contribution to the physical and chemical stability of the composition containing the cooling agent of the present invention, both during storage and use of the composition or any article containing such composition. Advantageously the presence of an ester derivative as described herein in the compositions herein helps solubilisation of the cooling agent into the compositions.

Highly preferred herein as the cooling agents are the cyclohexyl derivatives mentioned herein, preferably menthyl esters like menthyl lactate.

In one aspect of the present invention the compositions comprise as the cooling agent (a) a ketal, carboxamide, cyclohexanol derivative as described in claim 1 and/or cyclohexyl derivative as described in claim 1 , or a mixture thereof, in combination with (b) menthol and/or peppermint oil. Such compositions result in further optimized freshness sensation.

The compositions herein find application in any topical application, this term being taken in its broadest possible sense.

### Background art of the Invention

Cooling agent-containing compositions as well as their application in edible compositions like beverages and chewing-gum, in cosmetic products like shave lotions, deodorants, face creams, shampoos, toilet soaps and dentifrices are known. See for example US 5 451 404, US 5 266 592, US 5 756 857, US 4 136 163, US 4 230 688 or DE 26 08 226.

In U.S. Patent No's. 5,649,914 and 5,797,892; a toilet training aid is disclosed which generates a heating or cooling effect in the presence of urine from the wearer within the article. The heating or cooling effect is intended to cause the wearer discomfort in an attempt to aid in the toilet training process. This heating or cooling effect performs no useful function upon the article itself. Instead, the toilet training aid acts upon the wearer to cause the wearer to take some action (i.e., remove the wet article and apply a new one). Further, the toilet training aid responds solely to conditions within the article itself, not to conditions between the article and the wearer. Further, the toilet training aid is only functioning for a short period of time and is not designed to provide a sustained reduction in relative humidity or temperature for typical wear times. EP 704 195 discloses sanitary napkins to be used as menstrual detector containing a temperature-sensitive reactive chemical. Example of such temperature-sensitive reactive chemical include sodium thiosulfate or sodium hyposulfite, which can respond by turning cold upon coming into contact with and dissolving in a hot liquid, such as a menstrual flow.

None of these prior art references discloses nor suggests compositions comprising a cooling agent together with an ester derivative as described herein. Advantageously these compositions deliver optimized freshness sensation (i.e., more immediate and more long lasting freshness sensation as compared to same compositions in absence of said ester derivative), this while maintaining or even improving health of the external body surface to which the composition is applied and deodorizing the external body surface.

### Summary of the Invention

The present invention also encompasses hygienic articles, preferably hygienic disposable absorbent articles comprising such a composition.

### Detailed description of the Invention

As used herein the term, 'maintain skin health' means to preserve the natural state of healthy skin. The term 'improve skin health' refers to a reduction in the extent of adverse skin effects. These terms describe skin health in the area contacted by the compositions. It will be recognized that the compositions of the present invention when applied directly or indirectly to the skin and/or mucosal surface maintain or even improve skin health.

The term "disposable" article is used herein to describe articles that are not intended to be launched or otherwise restored or reused as an article (i.e., they are intended to be discarded after a single use and, preferably to be recycled, composted or otherwise disposed of in an environmentally compatible manner).

As used herein, the term 'hygienic article' refers to various articles of comfort and/or medical use, for the use by babies and adults or even animals.

The term "absorbent article" is used herein in a very broad sense including any article able to receive and/or absorb and/or contain and/or retain fluids and/or exudates, especially body fluids/body exudates. "Absorbent articles" as referred to herein include, without to be limited to, sanitary napkins, panty liners, incontinence pads, interlabial pads, breast pads, sweat-absorbent underarm pads, collar inserts, baby diapers, adult incontinence diapers, and human waste management devices. Typically such human urine or faecal management devices comprise a bag having an aperture and a flange surrounding the aperture for preferably adhesive attachment to the urogenital area and/or the perianal area of a wearer. Any faecal or urine management device known in the art is suitable for use herein. Such devices are described in for example in WO 99/00084 to WO 99/00092.

As used herein, the term 'body fluids and/or body exudates' refers to any fluids/exudates produced by human body occurring naturally or accidentally like for instance in the case of skin cutting, including for instance perspiration, urine, menstrual fluids, faeces, vaginal secretions and the like.

By the term 'topical application' or 'topical(ly) applied', as used herein, is meant directly laying on or spreading on epidermal tissue, especially outer skin, and/or mucosal surface of mammal, especially human.

The compositions of the invention are applied to hygienic articles to be contacted with skin and/or mucosal surfaces. Such articles are disposable absorbent articles such as diapers, incontinent pads, training pants, sanitary napkins, panty liners, tampons, interlabial pads and the like.

The present invention deals with (disposable) hygienic (absorbent) articles incorporating the compositions of the invention for conveying optimized freshness sensation to the user, while maintaining or even improving health of the skin and/or mucosal surface to which the compositions (or articles comprising the compositions) is topically applied to and deodorizing said skin and/or mucosal surface.

### Cooling agent

According to the present invention the composition comprise as an essential component a cooling agent or a mixture thereof.

By 'cooling agent' it is meant herein any agent able to convey to a mammal, preferably human, a freshness sensation (also called herein cooling sensation), when topically applied to said mammal/human.

Cooling agents for use herein are those for which the cooling effect (also called herein freshness effect) is a physiological effect due to the direct action of these agents on the nerve endings of the mammal body responsible for the detection of hot or cold without any occurrence of temperature change on the surface of the mammal body. It is believed that these agents act as a direct stimulus on the cold receptors at the nerve endings, which in turn stimulate the central nervous system. In this way a freshness/cooling sensation is simulated even in absence of real change in skin temperature. Due to the persistence of the stimuli a long lasting freshness/cooling sensation is delivered even after removal of the cooling agent.

It is to be understood herein that the freshness/cooling sensation is personnel to a given individual. It must be admitted that skin tests are somewhat subjective, some individuals experiencing a greater or lesser freshness sensation than others when subjected to the same test. The freshness perception depends on the density of thermo-receptors on skin and on the skin thickness. Typically it is observed that the thinner the skin is the more intense is the freshness sensation. Without to be bound by any theory, it is believed that the thinner the skin is, the more rapid is the penetration of the cooling agent through the skin and higher is the absorption level thereof. Further more studies have demonstrated that geographic factors and/or races further play a role in freshness perception.

Studies performed on cooling agent activity have showed that four features of the molecular structure of the cooling agents are particularly important to deliver freshness/cooling sensation. Reference is made to H.R. Watson et al., Journal of the Society of Cosmetic Chemist, Vol. 29, p185-200, 1978.

Suitable cooling agents for use herein posses the following properties:
- a hydrogen binding function - The cooling agents apparently need to have an atom or group able to bind hydrogen. The stronger the molecule's hydrogen binding capacity, the stronger the cooling effect. However, the presence of more than one hydrogen-binding group in its molecule can reduce its cooling effect, as the molecule would no longer have the correct lipophilic characteristics;
- a compact hydrocarbon skeleton such that the body's receptor is able to 'recognize' them;
- a balance between their hydrophilic and hydrophobic parts for both delivering cooling properties and able them to penetrate the biological membrane such as outer skin layers- The most common method of determining this balance is to use the Hansch log P value - the coefficient of water and n-octanol distribution according to Hansch. The log P value is acknowledged as being a crucial factor in a substance's pharmacological activity - especially as regards how it is transported through skin. The log p values of preferred cooling agents for use herein generally lies somewhere between 2.0 and 3.0;
- typically a molecular weight of between 150 and 350.
   Without to be bound by theory, it is speculated that such cooling agents are able to penetrate through the skin surface and depolarize (clear the potential differential between the inside and outside nervous cell membranes by blocking calcium ion exchange) the membrane of cold receptors. The cold perception is the result of the depolarization.
   More particularly, it is believed that due to binding calcium properties of the cooling agents, the equilibrium between the concentration of calcium ion outside and inside the nervous cell membrane is disturbed. In other words by reducing the calcium ion level outside the nervous cell membrane, the membrane is depolarized, resulting thereby in increased discharge rate of nerve fibers and hence transfer of electrical stimuli to central nervous system.
   Without to be bound by any theory, it is believed that the long lasting effect is linked to binding stability properties of the cooling agent and calcium ion complex. The higher is the stability of the complex cooling agent-calcium ion, the longer the calcium is linked to the cooling agent, the longer is the resulting freshness sensation.
   Suitable cooling agents for use herein are accordingly those that upon contact with skin/mucosal surface are able to stimulate the thermo-receptors of skin and/or mucosal surface to convey freshness sensation, without the need to modify temperature of skin/mucosal surface. Such cooling agents include, without to be limited thereto, peppermint oil, oil, tea tree oil, borneol, salicylate, ketals, carboxamides, cyclohexanol derivatives and/or cyclohexyl derivatives, as defined thereafter.

### Ketals:

Ketals suitable for use herein are according to the following formula: 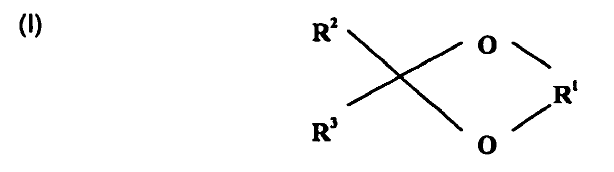 in which R¹ represents a C₂ -C₆ -alkylene radical having at least 1, but not more than 3, hydroxyl group(s), preferably one hydroxyl group, and either R² and R³ independently of one another represent C₁-C₁₀ -alkyl which is optionally substituted by 1 to 3 radicals selected from the group comprising hydroxyl, amino and halogen (such as fluorine, chlorine, bromine or iodine), C₅-C₇ -cycloalkyl, preferably cyclohexyl, C₆ -C₁₂ -aryl, preferably phenyl, with the proviso that the total of the C atoms of R² and R³ is not less than 3, or R² and R³ together represent an alkylene radical which, together with the carbon atom which carries the radicals R² and R³, forms a 5-7-membered ring, it being possible for this alkylene radical, in turn, to be substituted by C₁ -C₆ -alkyl groups.

Preferred radicals R² and R³ comprise methyl, isopropyl and tert-butyl.

The length of the radicals R² and R³ influences the effect of the compounds I: shorter radicals lead to an immediate, short effect; longer radicals lead to a delayed, but prolonged effect. When solubility of the compounds in water is desired it is preferable to use the compounds with short radicals R² and R³.

Preferred radicals R¹ embrace 1,2- and 1,3-alkylene radicals, which, together with the two oxygen atoms and with the carbon atom to which the two oxygen atoms are attached, form a dioxolane or dioxane ring.

Preferred compounds I in which R² and R³ together represent an alkylene radical are those of the formula 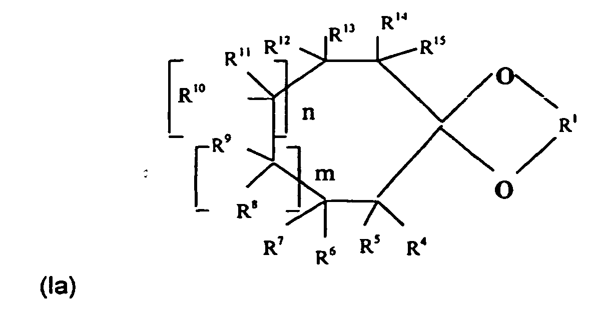 in which R⁴ to R¹⁵ independently of one another denote hydrogen or C₁ -C₆ -alkyl, preferably hydrogen or C₁ -C₄ -alkyl, and m and n independently of one another denote zero or 1.

Preferred compounds of the formula la are those in which the total of m+n is 1, i.e. ketals of an optionally substituted cyclohexanone.

Preferred substituents, of which there may be present, in particular, 1 to 3, are methyl, isopropyl and tert.-butyl.
The ketals I can be prepared by known processes. For example, ketal I will generally be prepared by acid-catalysed reaction of the ketone on which ketal I is based and not less than the equivalent amount of aliphatic C₃ -C₆ -alcohol having not less than 3 and not more than 5, preferably 3, hydroxyl groups. In general, the ketone on which ketal I is based and not less than 0.5 tool equivalents, but, as a rule, a 1.2- to 4-fold, preferably 1.5- to 3-fold excess of this amount of the C₃ -C₆ - alcohol having 3 to 5 hydroxyl groups will be employed. Examples of acid catalysts, which can be used, are p-toluenesulphonic acid, phosphoric acid or potassium hydrogen sulphate in catalytically effective amounts (for example 0.1 to 3 g of p-toluenesuphonic acid per mole of ketone). The reaction will preferably be carried out either in an organic solvent, which together with water forms an azeotrope, so that the water, which is liberated during the formation of the ketal, can be eliminated by azeotropic entrainment, or water-consuming coreagents such as, for example, trialkyl ortho esters are used. Examples of preferred organic solvents comprise benzene, toluene, xylene, chloroform, methylene chloride and trichloroethylene.

The reaction can be regarded as complete when water no longer separates out or when an ester/alcohol mixture is no longer separated out. It is recommended to wash the products subsequently with dilute alkali and with water, to separate and dry the organic phase, to strip off the solvent and, if appropriate, to purify the residue, for example by distillation.

Particularly preferred ketals I are those of the formulae 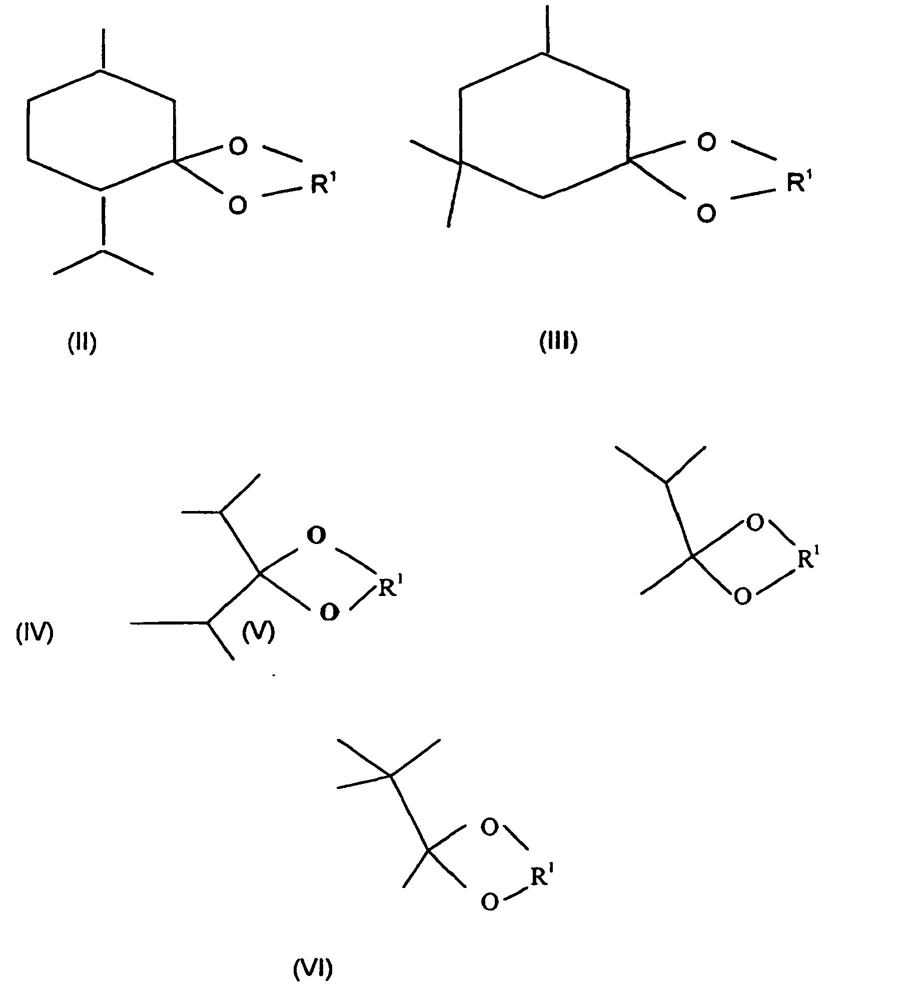 in which R¹ has the abovementioned meaning.

Particularly preferred ketals are the ketals II.

The ketals I to be employed herein can have asymmetric C atoms; optical isomerism can therefore occur. Depending on the starting material and the preparation methods used, they can exist in the form of mixtures of the optical isomers or in the form of pure isomers. The cooling effect of the isomers may differ, so that one or the other isomer may be preferred.

These ketals are for example described and exemplified in US 5 266 592,

An example of ketal commercially available include a ketal of formula (II) above, where R¹ is ethyl - (2 hydroxymethyl), namely menthone glycerol Ketal, available from Haarmann & Reimer GmbH (Germany) under the name Frescolat MGA®.

### Carboxamides

The carboxamides found most useful to be used herein are those described in U.S. Pat. No. 4,136,163, Jan. 23, 1979 to Wason et al., and U.S. Pat. No. 4,230,688, Oct. 28, 1980 to Rawsell et al.

Particularly suitable carboxamides for use herein are N-substituted-p-menthane3-carboxamides (US 4,136,163). These compounds are 3-substituted-p-menthanes of the formula: 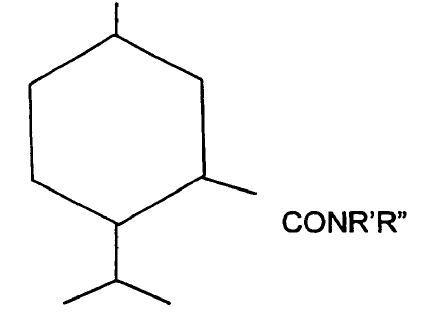 where R', when taken separately, is hydrogen or an aliphatic radical containing up to 25 carbon atoms; R" when taken separately is hydroxy, or an aliphatic radical containing up to 25 carbon atoms, with the proviso that when R' is hydrogen R" may also be an aryl radical of up to 10 carbon atoms and selected from the group consisting of substituted phenyl, phenalkyl or substituted phenalkyl, naphthyl and substituted naphthyl, pyridyl; and R' and R", when taken together with the nitrogen atom to which they are attached, represent a cyclic or heterocyclic group of up to 25 carbon atoms, e.g. piperidino, morpholino etc.

In the above definitions "aliphatic" is intended to include any straight-chained, branched-chained or cyclic radical free or aromatic unsaturation, and thus embraces alkyl, cycloalkyl, alkenyl, cycloalkenyl, alkynyl, hydroxyalkyl, acyloxyalkyl, alkoxy, alkoxyalkyl, aminoalkyl, acylaminoalkyl, carboxyalkyl and similar combinations.

Typical values for R' and R" when aliphatic are methyl, ethyl, propyl, butyl, i sobutyl, n-decyl, cyclopropyl, cyclohexyl, cyclopentyl, cycloheptylmethyl, 2-hydroxyethyl, 3-hydroxy-n-propyl, 6-hydroxy-n-hexyl, 2-aminoethyl, 2-acetoxyethyl, 2-ethylcarboxyethyl, 4-hydroxybut-2-ynyl, carboxymethyl etc. When R" is aryl typical values are benzyl, naphthyl, 4-methoxyphenyl, 4-hydroxyphenyl, 4-methylphenyl, 3-hydroxy-4-methylphenyl, 4-fluorophenyl, 4-nitrophenyl, 2-hydroxynaphthyl, pyridyl.

Other suitable carboxamides for use herein are certain acyclic tertiary and secondary carboxamides disclosed in US 4,230,688. These have the structure 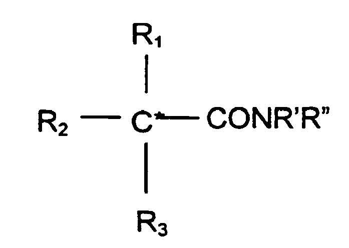 where R' and R", when taken separately, are each hydrogen, C₁-C₅ alkyl or C₁-C₈ hydroxyalkyl and provide a total of no more than 8 carbon atoms, with the proviso that when R' is hydrogen R" may also be alkylcarboxyalkyl of up to 6 carbon atoms;
R' and R", when taken together, represent an alkylene group of up to 6 carbon atoms, the opposite ends of which group are attached to the amide nitrogen atom thereby to form a nitrogen heterocycle, the carbon chain of which may optionally be interrupted by oxygen;
R₁ is hydrogen or C₁-C₅ alkyl; and R₂ and R₃ are each C₁-C₅ alkyl; with the provisos that (i) R₁, R₂ and R₃ together provide a total of at least 5 carbon atoms, preferably from 5-10 carbon atoms; and (ii) when R₁ is hydrogen, R₂ is C₂ -C₅ alkyl and R₃ is C₃-C₅ alkyl and at least one of R₂ and R₃ is branched, preferably in an alpha or beta position relative to the carbon atom marked (*) in the formula.

Where the compounds used have an asymmetric carbon atom either optical isomer may be used in pure form but generally a mixture of optical isomers will be used. In some cases the degree of cooling produced by the compounds on the skin will differ as between optical isomer, in which case one or other isomer may be preferred.

The preferred carboxamides used herein are the tertiary compounds, i.e., those wherein each of R₁, R₂ and R₃ is C₁-C₅ alkyl, especially those where R₁ is methyl, ethyl or n-propyl and at least one of R₂ and R₃ is a branched chain group having branching in an alpha or beta position relative to the C atom marked (*) in the formula. Also preferred are mon-substituted amides, i.e. where R' is H, and disubstituted amides where R' and R" are methyl or ethyl. A further preferred group consists of amides of the formula given where R₁ is hydrogen and at least one of R₂ and R₃ is branched in an alpha position relative to the carbon atom marked * in the formula.

The carboxamides may readily be prepared by conventional techniques, for example, by reaction of an acid chloride of the formula R₁ R₂ R₃ COCI with an amine of the formula HNR'R" in the presence of a hydrogen chloride acceptor. Such reactions are entirely conventional and the procedures involved will readily be understood by the persons skilled in the art.

Particularly suitable carboxamides for use herein are monosubstituted tertiary amides of the formula: 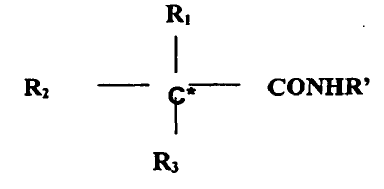 wherein R₁, R₂ and R₃ are each C₁-C₅ alkyl and together provide a total of at least 5, preferably 5-10 carbon atoms; and R' is C₁ -C₅ alkyl, C₁ -C₈ hydroxyalkyl or alkylcarboxyalkyl of up to 8 carbon atoms. In this group R₁ is preferably methyl, ethyl or n-propyl and one or both of R₂ and R₃ is branched in an alpha or beta position relative to the carbon atom marked (*).

An example of such aliphatic carboxamides is methyl-(N,2,3 tri-methyl)-2-isopropyl butanamide commercially available from IFF (International Flavors &Fragrances-US) under the name WS-23®. An example of such cyclic carboxamides is ethyl menthane carboxamide commercially available from Rhodia Chirex (UK) under the name WS-3®.

### Cyclohexanol derivatives

Suitable cyclohexanol derivatives for use herein are represented by the following general formula: 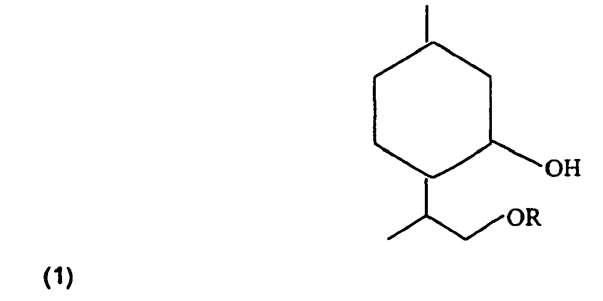 wherein R represents a linear or branched alkyl group having 1 to 5 carbon atoms.

The formal nomenclature thereof is 2-(2-alkoxy-1-methylethyl)-5-methylcyclohexanol. The above compound has a plurality of stereoisomers. Although any of them has strong refrigerating activity and is practically odorless, a cyclohexanol derivative represented by the following general formula: 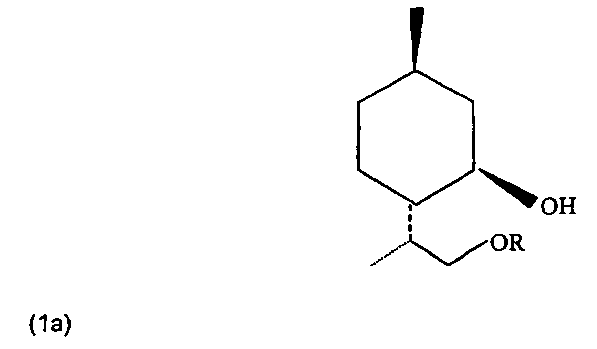 wherein R represents a linear or branched alkyl group having 1 to 5 carbon atoms, namely (1R, 2S, 5R, 8R)-2-(2-alkoxy-1-menthyflethyl)-5-methylcyclohexanol is preferred from the viewpoint of, for example, the continuity of cooling sensation.

Suitable cyclohexanol derivatives for use herein also include those of following general formula: 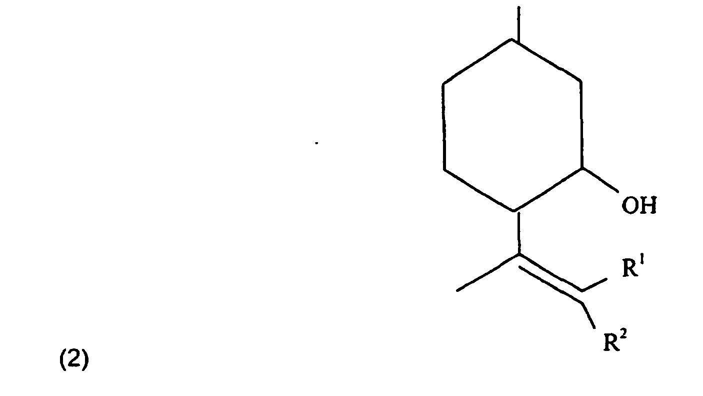 wherein R¹ and R² are independently hydrogen, or a linear or branched alkyl group having 1 to 5 carbon atom.

Examples of the linear or branched alkyl groups each having 1 to 5 carbon atoms represented by R in the above general formulae (1) and (1a) or by R¹ and R² in formulae (2), include methyl, ethyl, n-propyl, isopropyl, n-butyl, isobutyl, sec-butyl, tert-butyl, n-pentyl, isopentyl, sec-pentyl, tert-pentyl and neopentyl groups. Of these, methyl, ethyl, isopropyl, tert-butyl and n-pentyl groups are preferred, and a methyl group is especially preferred.

These compounds are described in more details including process of making them in US 5,756,857.

An example of commercially available cyclohexanol derivatives of formulae (2) above is 5-methyl-2- (1-methylethenyl) available from Takasago (Japan) under the name Coolact P®.

### Cyclohexyl derivatives

Suitable cyclohexyl derivatives for use herein are represented by the following general formula: 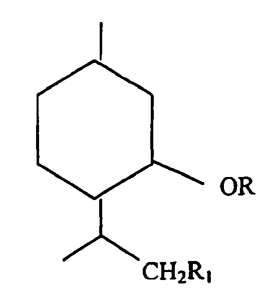 wherein R represents -H, a C₁-C₅ linear or branched alkyl group, alkenyl group, alkoxy group or acyloxy group, R₁ represents -H, or a linear or branched alkyl group having from 1 to 5 carbon atoms.

The above compound has a plurality of stereoisomers. Although any of them has strong refrigerating activity and is practically odorless, with the exception of those wherein both R and R₁ are hydrogen (i.e., menthol), cyclohexyl derivatives represented by the following general formula are preferred for use herein from the viewpoint of, for example, the continuity of cooling sensation.

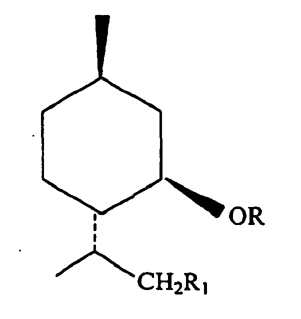

Highly preferred compounds are those of formula above wherein R₁=H and R is propanediol, carboxy-hydroxyethyl or carboxy hydroxypropyl. An example of these compounds is menthoxypropanediol available from Takasago under name TK10®

Even more preferred cyclohexyl derivative is menthyl lactate. Menthyl lactate is according to following formula

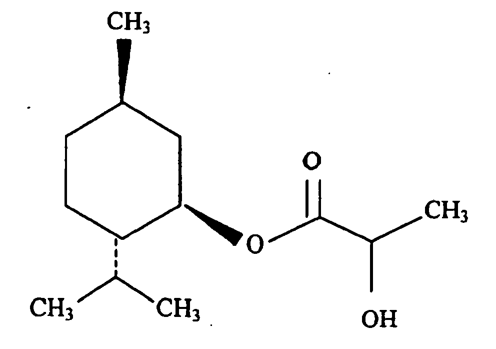

This compound is commercially available from Haarmann & Reimer GmbH (Germany) under the name FRESCOLAT, Type ML. It can also be readily made by processes known in the art by esterifying the hydroxy group of menthol with lactic acid. Two thirds of its molecular weight is attributable to the menthol moiety. Methyl lactate is virtually odorless, not suffering from the 'mint note' that otherwise customary in the case of menthol or peppermint oil. Advantageously this material has been found to be usefull as topical pain reliever (see WO96/14840).

The cooling agent might be use alone or in combination.

In one aspect of the present invention the cooling agents used are the cyclohexyl derivative of above-mentioned general formulae wherein both R and R₁ are hydrogen (i.e., menthol) and/or peppermint oil together with a second cooling agent. Menthol and/or peppermint oil is preferably not used alone herein as the cooling agent due to its strong mint odor and its tendency to irritate skin when used alone.

The second cooling agent is one of those listed herein above namely ketals, carboxamides, cyclohexanol derivatives as described in claim 1 and/or cyclohexyl derivatives as described in claim 1 with the exception of menthol. These later class of compound is preferred, especially menthyl lactate. Indeed beside the longer lasting freshness properties, the absence of mint odor and the excellent safety profile to skin associated with cyclohexyl derivatives (with the exception of menthol), menthyl lactate has also been found to act as pain reliever.

In one aspect of the present invention menthol is used together with a second cooling agent, preferably a cyclohexyl derivative as described in claim 1 with the exception of the cyclohexyl derivatives of above general formula mentioned herein wherein both R and R₁ are hydrogen (preferably menthyl lactate), or a ketal or a caboxamide, and/or cyclohexanol derivative as described in claim 1. Advantageously such compositions deliver optimized freshness sensation while keeping the mint odor at a level that is sufficiently low. Such compositions especially those wherein menthol and said second cooling agent are at a weight ratio of menthol to the second cooling agent in the range of 1/1 to 1/100, preferably 1/1 to 1/10, are such that the odor of the menthol is barely perceptible and are less irritating to skin and/or mucosal surface. Such compositions also deliver more optimized freshness properties due to the combined action of menthol, which is able to reach the thermo-receptors of the skin and/or mucosal surface immediately upon contact with skin, thereby fastening the perception of freshness, and due to the second agent like ketal, carboxamide, cyclohexyl derivative or cyclohexyl derivative with the exception of menthol, which all have a lower penetration rate (amount per time) through skin to reach the thermo-receptors than menthol, thereby resulting in longer lasting freshness properties.

The compositions of the present invention comprise an amount of the cooling agent or mixture thereof sufficient to convey freshness sensation to the person to which the composition is topically applied. As the degree and longevity of the freshness sensation vary from agents to agents, the amount of agents used in each compositions will vary widely.

Typically, the composition according to the present invention comprises the cooling agent or mixture thereof at a level of 0.1% to 99.9%, preferably from 3% to 90%, more preferably from 5% to 60%, and most preferably from 10% to 40% by weight of the composition.

### The ester derivatives

The compositions of the present invention comprise as an essential component an ester derivative of below mentioned formulae or mixture thereof.

In addition to their function as a vehicle for delivering an effective concentration of a cooling agent to a wearer's skin, the ester derivatives as described herein are particularly beneficial to skin, they improve skin hydratation and softness, and hence maintain or even improve skin health. The ester derivatives to be used herein assure a film-forming capacity on the skin, which gives emolliency and helps prevent skin dehydration when directly contacting the skin, thereby reducing or even eliminating the occurrence of skin itching or burning. The ester derivatives to be used herein are able to locate themselves between the layers of the epiderm (thanks to their similarity with substances naturally contained in the epiderm (stratum corneum)), enhancing thereby the elastic properties of the skin.

The presence of the ester derivatives as described herein after (preferably those of formulae (II) herein below), results in optimum freshness profile during wearing of the articles. The freshness sensation is not only delivered more rapidly upon topical application of the composition of the present invention but is also sustained over longer periods of time, as compared to topical application of the same composition with same type and level of cooling agent but in absence of such ester derivatives. Without to be bound by any theory, it is believed that the presence of the ester derivative solubilises the cooling agent helping it to penetrate more quickly through the outer layer(s) of the skin and/or mucosal surface to make it readily available to the thermo-receptors. Furthermore the ester derivative due to its high affinity to skin and/or mucosal surface, deposits on the skin and remains into contact and within the skin/mucous for prolonged periods of time, allowing thereby diffusion of the cooling agent through the skin upon prolonged periods of time, this results in further longer lasting freshness properties.

Advantageously, beside their emolliency properties, these ester derivatives, especially those according to formulae (II) below, can function as enzyme substrates, which, when acted upon by a hydrolyzing enzyme typically present in body fluid, will be hydrolyzed resulting in the release of free acids. The presence of these acids will lower the pH of the area where the esters are topically applied to. This will amount to inactivation of all or most enzymes present in this area and resulting from contact of this area with body fluids/exudates, such as the lipase enzymes, protease enzymes and the like. This effect is relatively long lasting. In other words, the presence of such esters not only provides optimized perception profile (including delivery of not only faster but also sustained/controlled freshness sensation), but also reduces or even prevents the occurrence of skin irritation or skin rash as well as the formation of malodor due to microbial activity. Indeed the use of these esters in the compositions of the present invention provides compositions with the additional benefit of deodorancy effect on the skin/mucosal area/surface.

Suitable ester derivatives for use herein are those according to the following formulae: 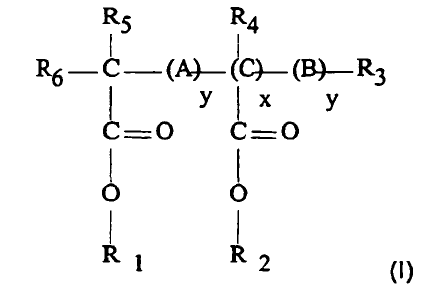 wherein R₃, R₄, R₅ and R₆ are independently an alkyl, alkenyl, arylalkyl, hydroxyalkyl, alkoxy groups of from 1 to 24 carbon atoms, hydroxy group or hydrogen group; R₁ and R₂ are independently an alkyl, alkenyl, arylalkyl, hydroxyalkyl, alkoxy groups of from 2 to 24 carbon atoms, hydroxy group or hydrogen group; A and B are independently a C₁-C₆ linear or branched alkylene, alkyl, alkenylene, alkoxylene, alkoxyl, hydroxyalkylene, hydroxyalkyl groups; the values of x are independently from 0 to 15; the values of y are independently 0 or 1.

Preferred ester compounds of formula (I) as defined above are those wherein x is 0, y is independently 0 or 1; R₁ is a C₁-C₅ alkyl group or hydrogen group; R₃, R₅ and R₆ are hydrogen, A and B are independently C₁-C₁₅ alkyl group.

It is even more preferred is methyl ester of stearic acid commercially available from Atofina.

Another particularly suitable ester derivatives for use herein are those of the formula: 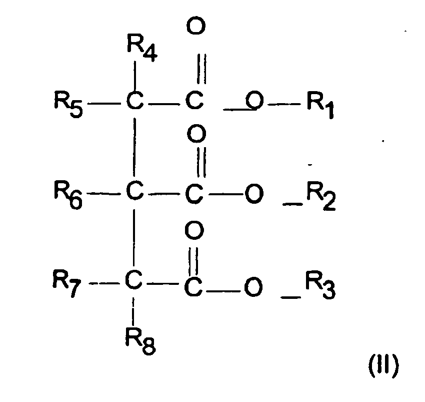 wherein R₁, R₂ and R₃ are independently an acyl, alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, acyl, alkenyl, hydroxyalkyl or alkoxy groups, hydroxy, chloride, bromide, amine or hydrogen.

Highly preferred are the compounds above wherein R₄, R₅, R₇ and R₈ of said compound are hydrogen, R₆ is hydrogen, hydroxy, C₁-C₄ linear or branched alkyl, alkenyl, hydroxyalkyl or alkoxy groups and preferably wherein R₁, R₂ and R₃ are independently a C₁-C₄ alkyl group or hydrogen.

Such preferred compounds include triethyl citrate, acetyl tributyl citrate, triacetyl citrate, O acetyl triethyl citrate. Highly preferred is triethyl citrate. Triethyl citrate is commercially available from Aldrich.

Typically the compositions according to the present invention comprise the ester derivatives or mixture thereof at a level from 99.9% to 0.1%, preferably from 97% to 10%, more preferably 95% to 40%, and most preferably from 90% to 60% by weight of the composition.

The compositions can be prepared by any conventional formulation technique known in the art.

The composition may be in a variety of forms, including, but not limited to, emulsions, dispersions, suspensions, gels, lotions, creams, oils, foams, ointments, powders, and the like. They may comprise any of the ingredients commonly used in the art for such compositions.

It is to be understood that the ingredients of the compositions of the present invention will depend on the character of the composition, thus lotions will generally comprise different additional ingredients than powders, as well as on the end used of the composition.

In the cosmetic creams, lotions, gels, oils or powders comprising the composition of the invention preferably an acidity source is present, preferably such that is capable to reduce the pH of the skin to below a pH of 8, more preferably below a pH of 7, more preferably below a pH of 6, or even more preferred below a pH of 5.

### Optional carriers

In formulating the compositions of the present invention the cooling agent and ester derivative as described herein might be used alone or in presence of additional conventional carriers which may be completely inert or which may be or contain other active ingredients.

A wide variety of carriers will be suitable, depending upon the end use of the compositions, such carriers including solids, liquids, emulsions, foams and gels. Typical carriers for use in the compositions include aqueous or alcoholic solutions, oils and fats such as hydrocarbon oils, fatty acid esters, long chain alcohols and silicone oils, finely divided solids such as starch or talc, emollients and the like.

Representative emollients useful in the present invention include, but are not limited to, emollients that are petroleum-based; sucrose ester fatty acids; polyethylene glycol and derivatives thereof; humectants; fatty acid ester type; alkyl ethoxylate type; fatty acid ester ethoxylates; fatty alcohol type; polysiloxane type; propylene glycol and derivatives thereof; glycerine and derivatives thereof, including glyceride, acetoglycerides, and ethoxylated glycerides of C₈-C₂₈ fatty acids; polyethylene glycol and derivatives thereof; propylene glycol; spermaceti or other waxes; fatty acids, particularly those having from 8 to 28 carbon atoms in their fatty chain such as mirytol; fatty alcohol ethers, particularly those having from 8 to 28 carbon atoms in their fatty chain, such as cetiol, stearic acid; propoxylated fatty alcohols; other fatty esters of polyhydroxy alcohols; lanolin and its derivatives; kaolin and its derivatives; sorbitol and its derivatives; trihydroxy stearin; or mixtures of these emollients.

The amount and type of any additional carrier like emollient in the composition of the present invention will depend on the character of the composition as well as on the end used of the composition.

### Optional odor control agents

Odour control agent or combinations thereof, known in the art for this purpose may be used in the compositions herein. These agents can typically be classified according to the type of odour the agent is intended to combat. Odors may be chemically classified as being acidic, basic or neutral.

Alternatively, the odor control agents may be categorized with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carboxylic acids such as citric acid, lauric acid, boric acid, adipic acid and maleic acid, oxidizing agents, antimicrobial agents, activated carbons, clays, zeolites, silicates, silica, diatomaceous earth and starches. Such odor control agents and systems are disclosed in more details hereinafter and for example in EP-A- 348 978, EP-A-510 619, WO 91/12029, WO 91/11977, WO 91/12030, WO 81/01643 and WO 96/06589. Highly preferred odor control agents are zeolite together with silicate and/or silica.

Alternative odor control agents are ion exchange resins such as those described in US 4 289 513 and US 3 340 875.

Masking agents such as perfumes may also be used as odor control agents herein.

Suitable odor control agents also include chelating agents and may be selected from amino carboxylates such as for example ethylenediamine- tetracetate, as described for example in US 4356190, amino phosphonates such as ethylenediaminetetrakis (methylene- phosphonates), polyfunctionally-substituted aromatic chelating agents as described in US 3 812 044 and mixtures thereof. Without intending to be bound by theory it is believed that the benefit of these materials is in part due to their exceptional ability to remove iron, copper, calcium, magnesium and manganese ions present in body fluids and their degradation products by the formation of chelates.

Another suitable odor control agent for use herein is an acidic pH buffer system, such as citric acid and sodium bicarbonate, sodium phosphate and sorbic acid buffer systems.

The amount and type of any additional odor control agent or mixture thereof in the composition of the present invention will depend on the character of the composition as well as on the end used of the composition.

### End Use Compositions

The compositions in which the cooling compositions find application are many and varied. These compositions include a wide variety of compositions for consumption by or application to the human body. Broadly speaking, these compositions can be classified as topical compositions, this term being taken in its broadest possible sense. Topical compositions include not only compositions such as perfumes, powders and other toiletries, lotions, liniments, oils and ointments applied to the external surfaces of the human body, whether for medical or other reasons, but also compositions applied to, or which, in normal usage, come in contact with internal mucous membranes of the body, such as those of the nose, mouth, or throat, whether by direct or indirect application or inhalation, and thus include nasal and throat sprays, dentifrice, mouthwash and gargle compositions. Also included within the present invention are toilet hygienic articles such as cleansing tissues, wipes, toilet papers, diapers, sanitary napkins, panty liners and the like, and toothpicks impregnated or coated with the compositions according to the present invention.

In a broad aspect of the invention the composition is applied on an article suitable to be contacted or even worn in direct contact with human body. Such articles include hygienic articles for use by babies, male or women like hygienic disposable absorbent articles.

The following illustrate the range of compositions into which the active cooling agent and ester derivative can be incorporated:

Toiletries including after shave lotions, shaving soaps, creams and foams, toilet water, deodorants and antiperspirants, "solid colognes", toilet soaps, bath oils and salts, shampoos, hair oils, talcum powders, face creams, hand creams, sunburn lotions, cleansing tissues, hygienic articles, dentifrices, toothpicks, mouthwashes, hair tonics, eyedrops.

Topical pharmaceuticals including antiseptic ointments, liniments, lotions, decongestants, counter-irritants, cough mixtures, throat lozenges, analgesics.

Because of their cooling effect on the skin and on the mucous membranes of the mouth, throat and nose the cooling compositions of the present invention may be used in a variety of topical pharmaceutical compositions, particularly where a counter-irritant is required.

Miscellaneous compositions such as water-soluble adhesive compositions for envelopes, postage stamps, adhesive labels etc.

Particular preparations according to the invention are discussed in more detail below.

Because of the cooling sensation imparted to the skin, a major utility of the composition herein will be in a wide range of toilet preparations and hygienic articles.

The particular preparations discussed below are to be taken as exemplary.

A major utility will be in after shave lotions, toilet water where the cooling compositions of the invention will be used in alcoholic or aqueous alcoholic solution, such solutions usually also containing a perfume or mild antiseptic or both.

Another field of utility will be in soaps, shampoos, bath oils where the cooling compositions will be used in combination with an oil or fat or a natural or synthetic surfactant e.g. a fatty acid salt or a lauroylsulphate salt, the composition usually also containing an essential oil or perfume. The range of soap compositions will include soaps of all kinds e.g. toilet soaps, shaving soaps, shaving foams.

A further class of toilet compositions includes cosmetic creams and additional emollients, such creams and emollients usually comprising a base emulsion and optionally a range of ingredients such as wax, preservative, perfume, antiseptics, astringents, pigments etc. Also included within this class are lipstick compositions, such compositions usually comprising an oil and wax base into which the cooling compositions of the invention can be incorporated along with the conventional ingredients, i.e. pigments, perfumes etc. Once again the formulation of such compositions is conventional.

Compositions for oral hygiene include mouthwash and dentifrice compositions. Dentifrice compositions may be of the powder, paste or liquid type and will usually comprise a finely divided abrasive or polishing material, e.g. precipitated chalk, silica, magnesium silicate, aluminum hydroxide or other similar materials well known in the art, and a detergent or foaming agent. Optional ingredients, which may also be included are flavoring agents and colorants, antiseptics, lubricants, thickeners, emulsifiers or plasticizers.

The present invention is preferably directed to hygienic disposable articles like bandages, thermal pads, acne pads, cold pads, wrist cooler, compresses, surgical pads/wound dressings, protective bedding covers, protective clothing, gloves, socks, pillow covers, protective face masks, ornamental/fashionable articles or eye wear, prothesis, plasters, wraps, hearing aids and the like; hygienic disposable articles for absorbing perspiration such as perspiration pads, underarm sweat pads, shoe insoles, shirt inserts, sporting clothes, cap inside liner and the like; hygienic disposable articles for animals like litters or animal waste management devices as well as hygienic disposable absorbent articles for use by babies and adults like panty liners, feminine napkins, incontinent pads, diapers, tampons, interlabial pads, dry or wet wipes, breast pads, human waste management devices and the like.

The compositions of the present invention can be comprised in a hygienic article preferably a disposable absorbent article. A particularly preferred absorbent article therefore is a wipe, a diaper, feminine protection article like panty liner, sanitary napkin or tampon. The diaper or feminine protection article preferably comprises the composition in the topsheet of the article, preferably on the surface of the article directly adjacent to the wearer, i.e., the so-called wearer-facing surface of the article.

The structure of the disposable absorbent article is not critical to the practice of the present invention.

The cooling composition of the present invention is incorporated into the hygienic article, preferably feminine protection article, in a sufficient amount so as to deliver the required freshness properties.

The disposable absorbent article, like feminine protection article, preferably contains the composition according to the present invention on at least a portion of the article such that the cooling agent or a mixture thereof is present at a level of from 0.01 gm⁻² to 300 gm⁻², preferably from 0.05 to 200 gm⁻², more preferably from 1 gm⁻² to 100 gm⁻² and most preferably from 2 gm⁻² to 20 gm⁻² per article.

A disposable hygienic article, typically an absorbent article generally comprises
- an absorbent core (which may consist of sub-structures);
- a fluid pervious topsheet;
- a fluid impervious backsheet.

The compositions of the present invention are preferably incorporated into a hygienic article, like feminine protection article, preferably into the topsheet structure. The composition may be incorporated onto the topsheet structure by diverse methods which will be readily apparent to those skilled in the art. For example, the composition can be, (optionally after being dispersed in aqueous or volatile carrier such as water, ethanol, or the like), applied to the topsheet, by spraying, dipping, printing, soaking or otherwise contacting the selected structural element of the hygienic article with the composition (optionally in presence of additional carrier), which is called herein impregnation.

### Topsheet

Generally, the topsheet is compliant, soft feeling, and non-irritating to the wearer's skin. Further, the topsheet is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibres (e.g., wood or cotton fibres), synthetic fibres (e.g., polyester or polypropylene fibres), or a combination of natural and synthetic fibres. There are a number of manufacturing techniques, which may be used to manufacture the topsheet. For example, the topsheet may be a nonwoven web of fibres spunbonded, carded, wet-laid, meltblown, hydroentangled, combinations of the above, or the like. The topsheet might comprise a single layer or several layers of material.

### Backsheet

In general the backsheet is compliant, flexible and soft feeling. The backsheet prevents the exudates absorbed and contained in the absorbent core from wetting clothes that contact the absorbent article such as undergarments. Preferably the backsheet is impervious to liquids (e.g., menses, sweat and/or urine). It can be manufactured from a thin plastic film, although other flexible liquid impervious materials can also be used. As used herein, the term "flexible" refers to materials that are compliant and will readily conform to the general shape and contours of the human body. The backsheet preferably also can have elastic characteristics allowing it to stretch in one or two directions.

The backsheet can comprise a woven or nonwoven material, polymeric films such i as thermoplastic films of polyethylene or polypropylene, or composite materials such as a film-coated nonwoven material or fiber coated film. Conventionally absorbent articles comprise a backsheet of a polyethylene film having a thickness of from about 0.012 mm to about 0.051 mm.

The backsheet is preferably breathable, i.e., allows the transmission of water vapor, or even more preferable the transmission of air, however without sacrificing its main purpose to provide leakage protection for absorbed liquids. The backsheet can also comprise more than one breathable layer so as to replace a single breathable backsheet layer by at least 2 or 3 layers of a different or the same material. In particular two breathable layers forming together the breathable backsheet are preferred.

The various core, topsheet and backsheet materials can be arranged in any way known in the art.

Also encompassed in the present invention is a process for making a hygienic article comprising the composition of the invention whereby the topsheet is impregnated with the composition before incorporation in the article.

The present invention is further illustrated by the following examples.

### Examples

### Example A

Cooling compositions were prepared as exemplified herein after by homogeneously mixing at room temperature (about 20°C) the herein after mentioned ingredients at indicated concentration:

### Composition 1

- 23 % by weight of menthyl lactate available from H&R (Germany) under the name Frescolat ML® together with 77% by weight of tri-ethyl citrate or tri-acetyl citrate (available from Aldrich)

### Composition 2

- 20 % by weight of menthoxypropanediol available from Takasago under the name TK-10® together with 80 % by weight of triethyl citrate or tri-acetyl citrate (available from Aldrich).

### Composition 3

- 20 % by weight of menthone glycerol ketal available from H&R under the name Frescolat MGA® and 80 % by weight of triethyl citrate (available from Aldrich).

### Composition 4

- 20% by weight of ethyl menthane carboxamide available from Givaudan Roure under the name WS3® and 80 % by weight of triethyl citrate (available from Aldrich).

### Composition 5

- 25 % by weight of menthyl lactate available from Haarman&Reimer (Germany) under the name Frescolat®ML, 5% by weight of I-Menthol available from Takasago under the name Menthol and 70 % by weight of triethyl citrate

### Composition 6

- 20 % by weight of menthyl lactate available from Haarman&Reimer (Germany) under the name Frescolat®ML, 10% by weight of I-Menthol available from Takasago under the name Menthol and 70 % by weight of triethyl citrate.

### Composition 7

- 23 % by weight of menthyl lactate available from H&R (Germany) under the name Frescolat ML® together with 77% by weight of methyl ester of stearic acid available from Atofina.

### Composition 8

- 20 % by weight of menthyl lactate available from Haarman & Reimer (Germany) under the name Frescolat®ML, 10% by weight of I-Menthol available from Takasago under the name Menthol and 70% by weight of methyl ester of stearic acid available from Atofina.

### Example B

Pantiliners were prepared by modifying panty liners commercially available, namely "Alldays"® manufactured by Procter & Gamble, Germany.

The topsheet is a film/non woven composite {film supplier code 45105 BP Chemical Plastic Germany, non woven supplier code T-27 AXC Corolind HDPE LINOTEC)

30 g/m² of an emollient-containing composition consisting of 23% by weight of menthyl lactate available from H&R (Germany) under the name Frescolat ML® and of 77% by weight of triehyl citrate (available from Aldrich) was sprayed homogenously over the whole surface of the wearer-facing surface of the topsheet.

The core is an airlaid material containing an absorbing gelling compound having a basis weight of 100 g/m² and available from Concert under the code GH 100.91209.

The backsheet comprises two layers a first layer and a second layer. The first layer (also called secondary backsheet) is in contact with the absorbent tissue and the second layer. The second layer is in contact with the first layer and the undergarment of the wearer. The first layer is a formed apertured film (HEX) {supplied by Tredegar Film Products B.V. Holland under the manufacturing code X-25368}. The second layer is composed of a microporous layer {supplied by EXXON Company IL under the manufacturing code EXXAIRE BF112 W}. Each backsheet layer is joined over the full surface by an extensively overlapped spiral glue application at a basis weight of approximately 8 g/m². The glue used for attachment of both backsheet layers was supplied by SAVARE' SpA. Italy (under the material code PM17).

These panty liners were found to improve comfort to the wearer in use, including quasi immediate (after only about 2 minutes of wearing time) and long lasting freshness sensation (over more than 3 to 4 hours), while maintaining or even improving skin health and deodorizing skin/mucosal surface.

## Claims

1. A kiaper or feminine protection article containing in the topsheet structure a composition comprising
(a) a cooling agent selected from the group consisting of
- ketals;
- carboxamides;
- cyclohexanol derivatives according to the following general formulae: 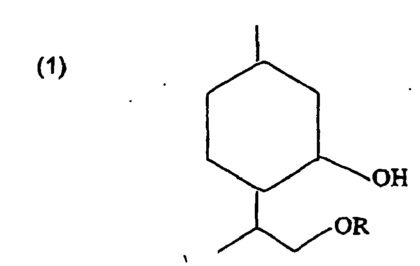
wherein R represents a linear or branched alkyl group having 1 to 5 carbon atoms, 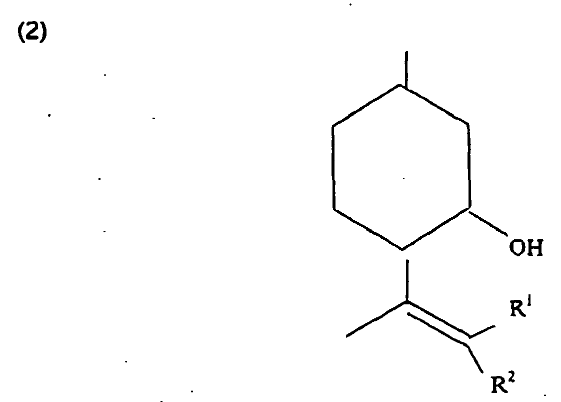 wherein R¹ and R² are independently hydrogen, or a linear or branched alkyl group having 1 to 5 carbon atom;
- cyclohexyl derivatives according to the following general formula 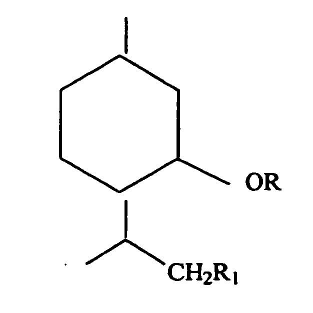
wherein R represents -H, a C₁-C₅ linear or branched alkyl group, a C₁-C₅ alkenyl group, a C₁-C₅ alkoxy group or a C₁-C₅ acyloxy group, R₁ represents -H, or a linear or branched alkyl group having from 1 to 5 carbon atoms, with the exception of those wherein both R and R1 are hydrogen or
wherein R₁=H and R is propanediol, carboxy-hydroxyethyl or carboxy hydroxypropyl;
- cyclohexyl derivative of the formula 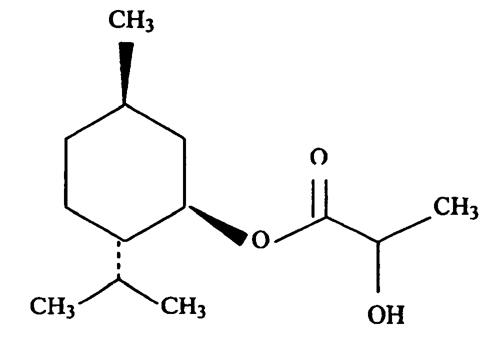
- borneol, tea tree oil, and mixtures thereof;
(b) together with an ester derivative of following formulae: 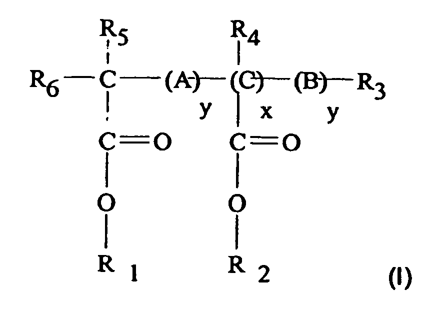
wherein R₁ and R₂ are independently an alkyl, alkenyl, arylalkyl, hydroxyalkyl, alkoxy groups of from 2 to 24 carbon atoms, hydroxy group or hydrogen group; R₃, R₄, R₅, and R₆ are independently an alkyl, alkenyl, arylalkyl, hydroxyalkyl, alkoxy groups of from 1 to 24 carbon atoms, hydroxy group or hydrogen group; A and B are independently a C₁-C₆ linear or branched alkylene, alkyl, alkenylene, alkoxylene, alkoxyl, hydroxyalkylene, hydroxyalkyl groups; the values of x are independently from 0 to 15; the values of y are independently 0 or 1,
or 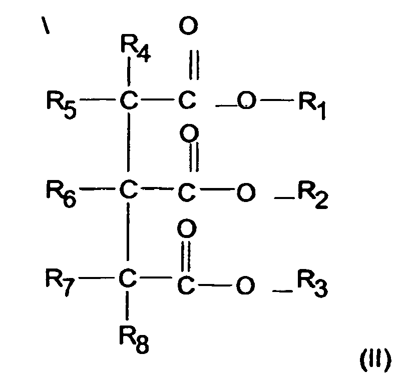 wherein R₁, R₂ and R₃ are independently an acyl, alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, acyl, alkenyl, hydroxyalkyl or alkoxy groups, hydroxy, chloride, bromide, amine or hydrogen, or mixture thereof.

2. A diaper or feminine protection article according to claim 1 wherein the cooling agent is selected from the group consisting of:
- a ketal according to the following formula: 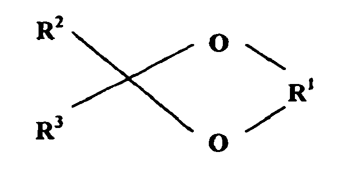 in which R¹ represents a C₂ -C₆ -alkylene radical having at least 1, but not more than 3, hydroxyl group(s), preferably 1 hydroxyl group, and either R² and R³ independently of one another represent C₁-C₁₀ -alkyl which is optionally substituted by 1 to 3 radicals selected from the group comprising hydroxyl, amino and halogen (such as fluorine, chlorine, bromine or iodine), C₅-C₇ -cycloalkyl, preferably cyclohexyl, C₆ -C₁₂ -aryl, preferably phenyl, with the proviso that the total of the C atoms of R² and R³ is not less than 3, or R² and R³ together represent an alkylene radical which, together with the carbon atom which carries the radicals R² and R³ , forms a 5-7-membered ring, it being possible for this alkylene radical, in turn, to be substituted by C₁ -C₆ -alkyl groups, or mixtures thereof;
- or a carboxamide of the following formula: 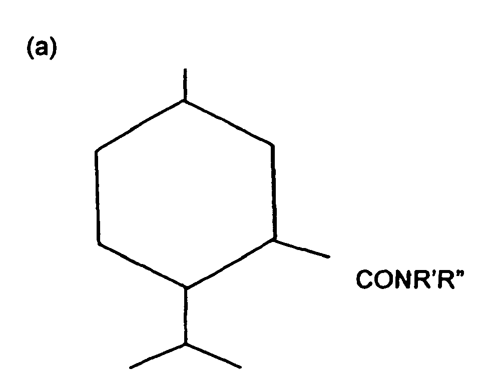 wherein R', when taken separately, is hydrogen or an aliphatic radical containing up to 25 carbon atoms; R" when taken separately is hydroxy, or an aliphatic radical containing up to 25 carbon atoms, with the proviso that when R' is hydrogen R" may also be an aryl radical of up to 10 carbon atoms and selected from the group consisting of substituted phenyl, phenalkyl or substituted phenalkyl, naphthyl and substituted naphthyl, pyridyl; and R' and R", when taken together with the nitrogen atom to which they are attached, represent a cyclic or heterocyclic group of up to 25 carbon atoms, or mixtures thereof,
or 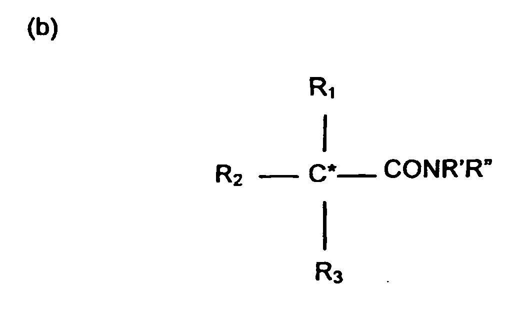 wherein R' and R", when taken separately, are each hydrogen, C₁-C₅ alkyl or C₁-C₈ hydroxyalkyl and provide a total of no more than 8 carbon atoms, with the proviso that when R' is hydrogen R" may also be alkylcarboxyalkyl of up to 6 carbon atoms; R' and R", when taken together, represent an alkylene group of up to 6 carbon atoms, the opposite ends of which group are attached to the amide nitrogen atom thereby to form a nitrogen heterocycle, the carbon chain of which may optionally be interrupted by oxygen; R₁ is hydrogen or C₁-C₅ alkyl; and R₂ and R₃ are each C₁-C₅ alkyl; with the provisos that (i) R₁, R₂ and R₃ together provide a total of at least 5 carbon atoms, preferably from 5-10 carbon atoms; and (ii) when R₁ is hydrogen, R₂ is C₂ -C₅ alkyl and R₃ is C₃-C₅ alkyl and at least one of R₂ and R₃ is branched, preferably in an alpha or beta position relative to the carbon atom marked (*) in the formula, or a mixture thereof;
- or a cyclohexanol derivative as defined in claim 1
- or a cyclohexyl derivative as defined in claim 1 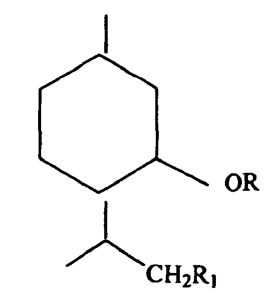
- or a mixture thereof.

3. A diaper or feminine protection article according to claim 1, wherein the cooling agent (a) is most preferably menthyl lactate; and further comprising a second cooling agent (b) being menthol and/or peppermint oil, the cooling agents being typically present in a weight ratio of (b) to (a) from 1/1 to 1/100.

4. A diaper or feminine protection article according to any of the preceding claims wherein the ester derivative is according to formulae (II) and preferably is triethyl citrate, acetyl tributyl citrate and/or triacetyl citrate.

5. A diaper or feminine protection article according to any of the preceding claims wherein the cooling agent or a mixture thereof is present at a level of 0.1% to 99.9% preferably from 3% to 90%, more preferably from 5% to 60%, and most preferably from 10% to 40% by weight of the total composition.

6. A diaper or feminine protection article according the any of the preceding claims wherein the ester derivative or a mixture thereof is present at a level of from 99.9% to 0.1%, preferably from 97% to 10%, more preferably 95% to 40%, and most preferably from 90% to 60% by weight of the total composition.

7. A feminine protection article according to any of the preceding claims, selected from a panty liner and a feminine napkin..

## Patentansprüche

1. Windel oder Damenhygieneartikel, die/der in der Oberschichtstruktur eine Zusammensetzung aufweist, die Folgendes umfasst:
(a) ein Kühlmittel, ausgewählt aus der Gruppe bestehend aus
- Ketalen;
- Carboxamiden;
- Cyclohexanolderivaten gemäß den folgenden allgemeinen Formeln: 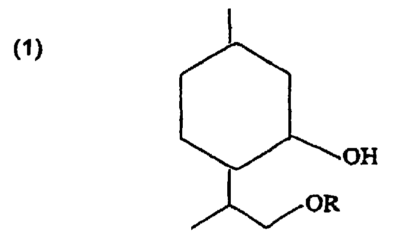 worin R für eine lineare oder verzweigte Alkylgruppe mit 1 bis 5 Kohlenstoffatomen steht, 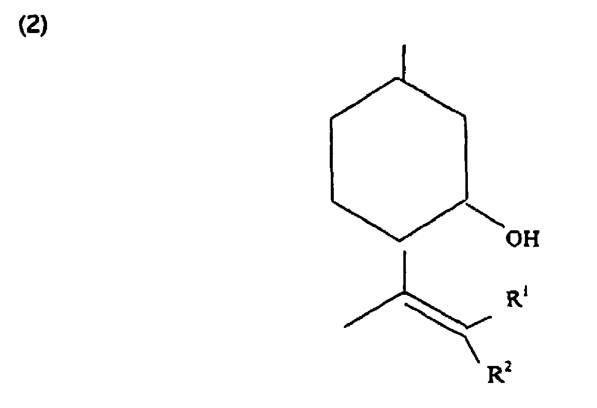 worin R¹ und R² unabhängig voneinander Wasserstoff oder eine lineare oder verzweigte Alkylgruppe mit 1 bis 5 Kohlenstoffatomen sind;
- Cyclohexylderivaten gemäß der folgenden allgemeinen Formel 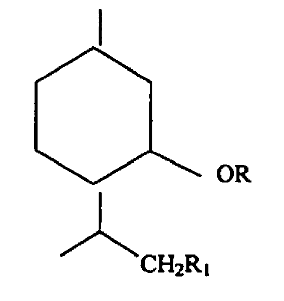 worin R für H, eine lineare oder verzweigte C₁-C₅-Alkylgruppe, eine C₁-C₅-Alkenylgruppe, eine C₁-C₅-Alkoxygruppe oder eine C₁-C₅-Acyloxygruppe steht, R₁ für H oder eine lineare oder verzweigte Alkylgruppe mit 1 bis 5 Kohlenstoffatomen steht, mit der Ausnahme derer, bei denen sowohl R als auch R1 Wasserstoff sind, oder
worin R₁=H und R Propandiol, Carboxyhydroxyethyl oder Carboxyhydroxypropyl ist;
- Cyclohexylderivat der Formel 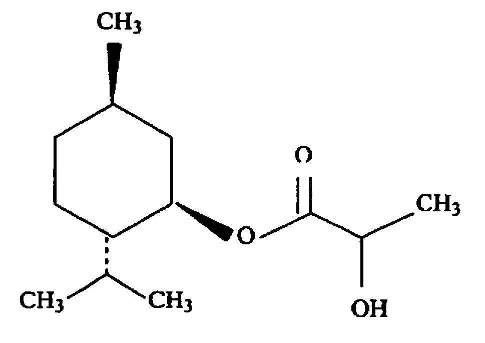
- Borneol, Teebaumöl und Mischungen davon;
(b) zusammen mit einem Esterderivat folgender Formeln: 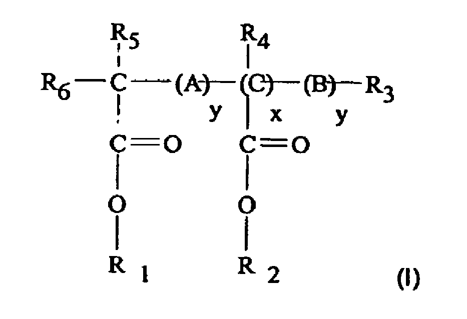 worin R₁ und R₂ unabhängig voneinander eine Alkyl-, Alkenyl-, Arylalkyl-, Hydroxyalkyl-, Alkoxygruppe mit 2 bis 24 Kohlenstoffatomen, Hydroxygruppe oder Wasserstoffgruppe sind; R₃, R₄, R₅ und R₆ unabhängig voneinander eine Alkyl-, Alkenyl-, Arylalkyl-, Hydroxyalkyl-, Alkoxygruppe mit 1 bis 24 Kohlenstoffatomen, Hydroxygruppe oder Wasserstoffgruppe sind; A und B unabhängig voneinander eine lineare oder verzweigte C₁-C₆-Alkylen-, -Alkyl-, -Alkenylen-, -Alkoxylen-, -Alkoxyl-, -Hydroxyalkylen-, -Hydroxyalkylgruppe sind;
die Werte von x unabhängig voneinander von 0 bis 15 betragen;
und die Werte von y unabhängig voneinander 0 oder 1 betragen,
oder 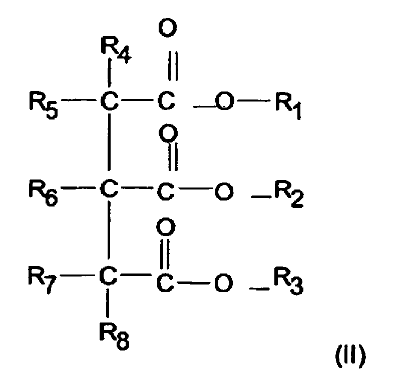 worin R₁, R₂ und R₃ unabhängig voneinander eine Acyl-, Alkyl- oder Alkenyl- oder Hydroxyalkylgruppe mit 1 bis 22 Kohlenstoffatomen sind und R₄, R₅, R₆, R₇ und R₈ unabhängig voneinander ausgewählt sind aus der Gruppe bestehend aus linearen oder verzweigten C₁-C₁₀-Alkyl-, -Acyl, -Alkenyl-, -Hydroxyalkyl- oder -Alkoxygruppen, Hydroxy, Chlorid, Bromid, Amin oder Wasserstoff oder Mischungen davon.

2. Windel oder Damenhygieneartikel nach Anspruch 1, wobei das Kühlmittel ausgewählt ist aus der Gruppe bestehend aus:
- einem Ketal gemäß folgender Formel: 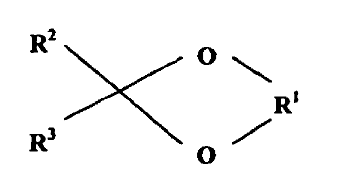 worin R¹ für einen C₂-C₆-Alkylenrest mit mindestens 1, jedoch nicht mehr als 3 Hydroxygruppe(n), vorzugsweise 1 Hydroxygruppe, steht und R² und R³ unabhängig voneinander stehen für C₁-C₁₀-Alkyl, das wahlweise mit 1 bis 3 Radikalen, ausgewählt aus der Gruppe, umfassend Hydroxyl, Amino und Halogen (wie z. B. Fluor, Chlor, Brom oder lod) substituiert ist, C₅-C₇-Cycloall, vorzugsweise Cyclohexyl, C₆-C₁₂-Aryl, vorzugsweise Phenyl, mit der Maßgabe, dass die Gesamtzahl der C-Atome von R² und R³ nicht kleiner als 3 ist oder R² und R³ zusammen für ein Alkylenradikal stehen, das zusammen mit dem Kohlenstoffatom, das die Radikale R² und R³ trägt, einen 5- bis 7-gliedrigen Ring bildet, wobei es wiederum bei diesem Alkylenradikal möglich ist, durch C₁-C₆-Alkylgruppen substituiert zu werden, oder Mischungen davon;
- oder einem Carboxamid der folgenden Formel: 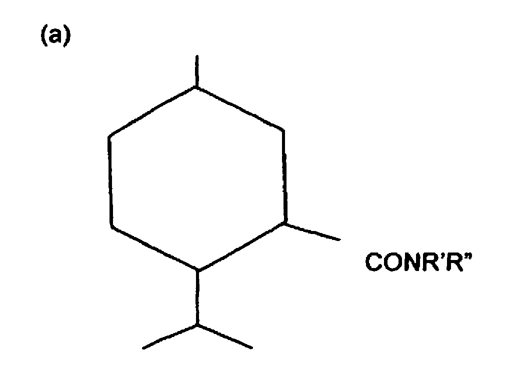 worin R', wenn es separat betrachtet wird, Wasserstoff oder ein aliphatisches Radikal mit bis zu 25 Kohlenstoffatomen ist; R", separat betrachtet, Hydroxy oder ein aliphatisches Radikal ist, das bis zu 25 Kohlenstoffatome enthält, mit der Maßgabe, dass R", wenn R' Wasserstoff ist, auch ein Arylradikal mit bis zu 10 Kohlenstoffatomen sein kann und aus der Gruppe bestehend aus substituiertem Phenyl, Phenalkyl oder substituiertem Phenalkyl, Naphthyl und substituiertem Naphthyl, Pyridyl ausgewählt sein kann;
und R' und R" gemeinsam mit dem Stickstoffatom betrachtet, an das sie gebunden sind, eine zyklische oder heterozyklische Gruppe mit bis zu 25 Kohlenstoffatomen oder Mischungen davon darstellen,
oder 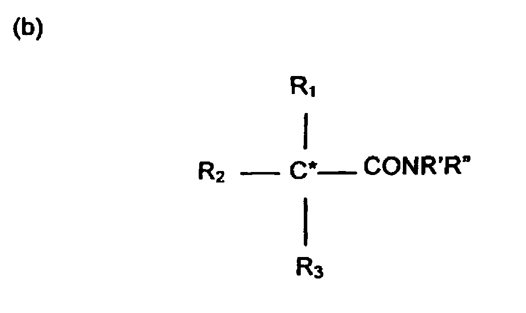 worin R' und R", wenn sie separat betrachtet werden, jeweils Wasserstoff, C₁-C₅-Alkyl oder C₁-C₈-Hydroxyalkyl sind und eine Gesamtzahl von nicht mehr als 8 Kohlenstoffatomen bereitstellen, mit der Maßgabe, dass, wenn R' Wasserstoff ist, R" auch Alkylcarboxyalkyl mit bis zu 6 Kohlenstoffatomen sein kann; R' und R", wenn sie gemeinsam betrachtet werden, für eine Alkylengruppe mit bis zu 6 Kohlenstoffatomen stehen, wobei die gegenüberliegenden Enden der Gruppe an das Stickstoffatom des Amids gebunden sind, um dadurch einen Stickstoffheterocyclus zu bilden, wobei die Kohlenstoffkette davon wahlweise durch Sauerstoff unterbrochen sein kann; R₁ Wasserstoff oder Cₗ-C₅-Alkyl ist; und R₂ und R₃ jeweils C₁-C₅-Alkyl sind; mit den Maßgaben, dass (i) R₁, R₂ und R₃ zusammen eine Gesamtzahl von mindestens 5 Kohlenstoffatomen, vorzugsweise von 5 bis 10 Kohlenstoffatomen bereitstellen; und (ii) wenn R₁ Wasserstoff ist, R₂ C₂-C₅-Alkyl ist und R₃ C₃-C₅-Alkyl ist und mindestens eines von R₂ und R₃ verzweigt ist, vorzugsweise in einer alpha- oder beta-Stellung bezüglich des in der Formel mit (*) markierten Kohlenstoffatoms, oder eine Mischung davon;
- oder einem Cyclohexanolderivat nach Anspruch 1
- oder einem Cyclohexylderivat nach Anspruch 1 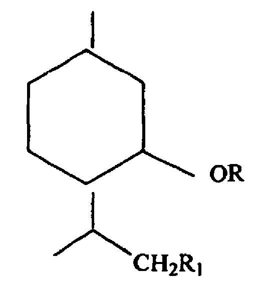
- oder einer Mischung davon.

3. Windel oder Damenhygieneartikel nach Anspruch 1, wobei das Kühlmittel (a) am meisten bevorzugt Menthyllactat ist; und ferner ein zweites Kühlmittel (b) umfassend, das Menthol und/oder Pfefferminzöl ist, wobei die Kühlmittel üblicherweise in einem Gewichtsverhältnis von (b) zu (a) von 1/1 bis 1/100 vorliegen.

4. Windel oder Damenhygieneartikel nach einem der vorstehenden Ansprüche, wobei das Esterderivat der Formel (II) entspricht und vorzugsweise Triethylcitrat, Acetyltributylcitrat und/oder Triacetylcitrat ist.

5. Windel oder Damenhygieneartikel nach einem der vorstehenden Ansprüche, wobei das Kühlmittel oder eine Mischung davon mit einem Gehalt von 0,1 Gew.-% bis 99,9 Gew.-%, vorzugsweise von 3 Gew.-% bis 90 Gew.-%, mehr bevorzugt von 5 Gew.-% bis 60 Gew.-% und am meisten bevorzugt von 10 Gew.-% bis 40 Gew.-% der Gesamtzusammensetzung vorliegt.

6. Windel oder Damenhygieneartikel nach einem der vorstehenden Ansprüche, wobei das Esterderivat oder eine Mischung davon mit einem Gehalt von 99,9 Gew.-% bis 0,1 Gew.-%, vorzugsweise von 97 Gew.-% bis 10 Gew.-%, mehr bevorzugt von 95 Gew.-% bis 40 Gew.-% und am meisten bevorzugt von 90 Gew.-% bis 60 Gew.-% der Gesamtzusammensetzung vorliegt.

7. Damenhygieneartikel nach einem der vorstehenden Ansprüche, ausgewählt aus einer Slipeinlage und einer Damenbinde.

## Revendications

1. Couche ou article de protection féminine contenant dans la structure de feuille de dessus une composition comprenant
(a) un agent de refroidissement choisi dans le groupe constitué de
- cétals ;
- carboxamides ;
- dérivés de cyclohexanol selon les formules générales suivantes : 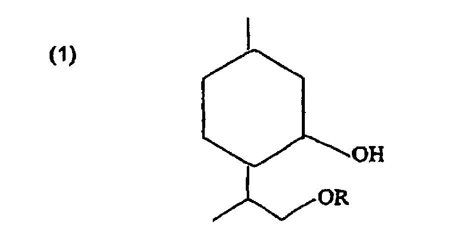 dans laquelle R représente un groupe alkyle linéaire ou ramifié ayant 1 à 5 atomes de carbone, 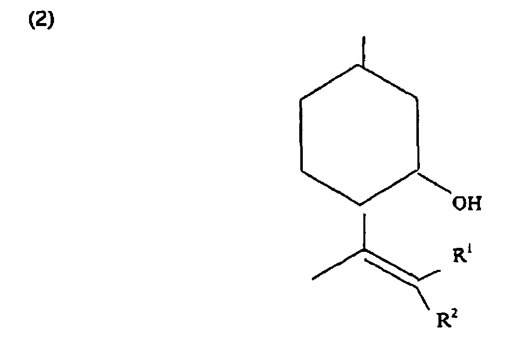 dans laquelle R¹ et R² sont indépendamment un hydrogène ou un groupe alkyle linéaire ou ramifié ayant 1 à 5 atomes de carbone ;
- dérivés de cyclohexyle selon la formule générale suivante 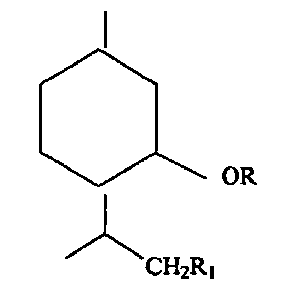 dans laquelle R représente H, un groupe alkyle linéaire ou ramifié en C₁ à C₅, un groupe alcényle en C₁ à C₅, un groupe alcoxy en C₁ à C₅ ou un groupe acyloxy en C₁ à C₅, R₁ représente H ou un groupe alkyle linéaire ou ramifié ayant de 1 à 5 atomes de carbone, à l'exception de ceux où tant R que R₁ sont un hydrogène ou
dans laquelle R₁=H et R est un propane-diol, un carboxy-hydroxyéthyle ou un carboxy hydroxypropyle ;
- dérivés de cyclohexyle de formule 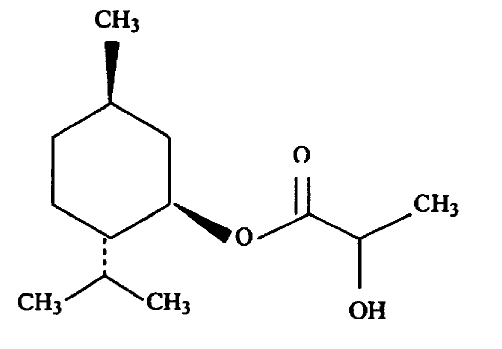
- bornéol, huile de théier, et leurs mélanges ;
(b) conjointement avec un dérivé d'ester de formules suivantes : 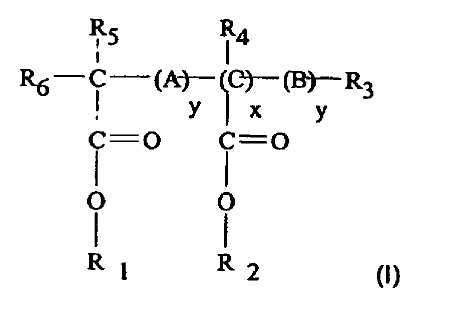 dans laquelle R₁ et R₂ sont indépendamment des groupes alkyle, alcényle, arylalkyle, hydroxyalkyle, alcoxy allant de 2 à 24 atomes de carbone, un groupe hydroxy ou un groupe hydrogène ; R₃, R₄, R₅ et R₆ sont indépendamment des groupes alkyle, alcényle, arylalkyle, hydroxyalkyle, alcoxy allant de 1 à 24 atomes de carbone, un groupe hydroxy ou un groupe hydrogène ; A et B sont indépendamment des groupes linéaires ou ramifiés alkylène, alkyle, alcénylène, alcoxylène, alcoxyle, hydroxyalkylène, hydroxyalkyle en C₁ à C₆ ; les valeurs de x sont indépendamment de 0 à 15 ; les valeurs de y sont indépendamment 0 ou 1
ou 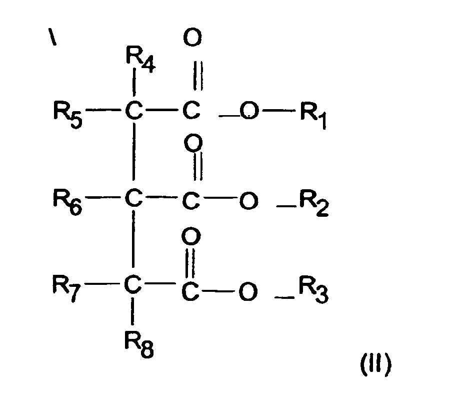 dans laquelle R₁, R₂ et R₃ sont indépendamment un groupe acyle, alkyle ou alcényle ou hydroxyalkyle avec de 1 à 22 atomes de carbone et R₄, R₅, R₆, R₇ et R₈ sont indépendamment choisis dans le groupe constitué de groupes linéaires ou ramifiés alkyle, acyle, alcényle, hydroxyalkyle ou alcoxy en C₁ à C₁₀, un hydroxy, un chlorure, un brome, une amine ou un hydrogène ou leur mélange.

2. Couche ou article de protection féminine selon la revendication 1, où l'agent de refroidissement est choisi dans le groupe constitué de :
- un cétal selon la formule suivante : 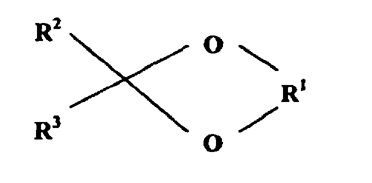 dans laquelle R¹ représente un radical alkylène en C₂ à C₆ ayant au moins 1, mais pas plus de 3, groupe(s) hydroxyle, de préférence 1 groupe hydroxyle et soit R² et R³ indépendamment l'un de l'autre, représentent un alkyle en C₁ à C₁₀ qui est facultativement substitué par 1 à 3 radicaux choisis dans le groupe comprenant un hydroxyle, une amine et un halogène (tel que le fluor, le chlore, le brome ou l'iode), un cycloalkyle en C₅ à C₇, de préférence un cyclohexyle, un aryle en C₆ à C₁₂, de préférence du phényle, à condition que le total des atomes C de R² et R³ ne soit pas inférieur à 3, soit R² et R³ ensemble représentent un radical alkylène qui, conjointement avec l'atome de carbone qui porte les radicaux R² et R³, forme un cycle de 5 à 7 chaînons, avec la possibilité que ce radical alkylène, à son tour, soit substitué par des groupes alkyle en C₁ à C₆ ou des mélanges de ceux-ci ;
- ou un carboxamide de formule suivante : 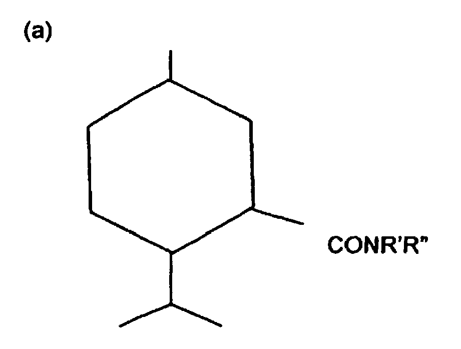 dans laquelle R', lorsqu'il est pris séparément, est un hydrogène ou un radical aliphatique contenant jusqu'à 25 atomes de carbone ; R", lorsqu'il est pris séparément, est un hydroxy ou un radical aliphatique contenant jusqu'à 25 atomes de carbone, à condition que lorsque R' est un hydrogène, R" puisse également être un radical aryle allant jusqu'à 10 atomes de carbone et choisi dans le groupe constitué de phényle substitué, phénalkyle ou phénalkyle substitué, naphtyle et naphtyle substitué, pyridyle ; et R' et R", lorsqu'ils sont pris ensemble avec l'atome d'azote auquel ils sont fixés, représentent un groupe cyclique ou hétérocyclique allant jusqu'à 25 atomes de carbone ou leurs mélanges ;
ou 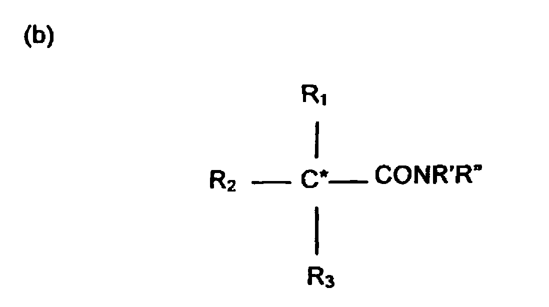 dans laquelle R' et R", lorsqu'ils sont pris séparément, sont chacun de l'hydrogène, un alkyle en C₁ à C₅ ou un hydroxyalkyle en C₁ à C₈ et fournissent un total non supérieur à 8 atomes de carbone, à condition que que lorsque R' est l'hydrogène, R" puisse également être un alkylcarboxyalkyle de jusqu'à 6 atomes de carbone ; R' et R", lorsqu'ils sont pris ensemble, représentent un groupe alkylène de jusqu'à 6 atomes de carbone, groupe dont les extrémités opposées sont fixées à l'atome d'azote de l'amide, afin de former ainsi un hétérocycle d'azote, dont la chaîne de carbone peut, facultativement, être interrompue par l'oxygène ; R₁ est un hydrogène ou un alkyle en C₁ à C₅ ; et R₂ et R₃ sont chacun un alkyle en C₁ à C₅ ; à condition que (i) R₁, R₂ et R₃ fournissent ensemble un total d'au moins 5 atomes de carbone, de préférence entre 5 et 10 atomes de carbone ; et (ii) lorsque R₁ est de l'hydrogène, R₂ est un alkyle en C₂ à C₅ et R₃ est un alkyle en C₃ à C₅ et au moins un groupe parmi R₂ et R₃ est ramifié, de préférence en une position alpha ou bêta par rapport à l'atome de carbone marqué (*) dans la formule ou un mélange de ceux-ci ;
- ou un dérivé de cyclohexanol comme défini dans la revendication 1
- ou un dérivé de cyclohexyle comme défini dans la revendication 1 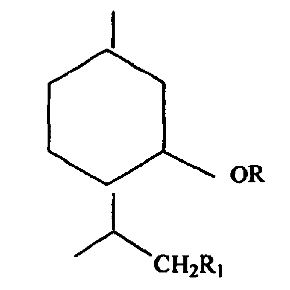
- ou un de leurs mélanges.

3. Couche ou article de protection féminine selon la revendication 1, où l'agent de refroidissement (a) est le plus préférablement du lactate de menthyle ; et comprenant, en outre, un deuxième agent de refroidissement (b) qui est du menthol et/ou de l'essence de menthe poivrée, les agents de refroidissement étant typiquement présents dans des rapports pondéraux de (b) sur (a) de 1:1 à 1:100.

4. Couche ou article de protection féminine selon l'une quelconque des revendications précédentes, où le dérivé d'ester est selon les formules (II) et de préférence est du citrate de triéthyle, du citrate d'acétyl-tributyle et/ou du citrate de triacétyle.

5. Couche ou article de protection féminine selon l'une quelconque des revendications précédentes, où l'agent de refroidissement ou un mélange de celui-ci est présent à un taux de 0,1 % à 99,9 %, de préférence de 3 % à 90 %, plus préférablement de 5 % à 60 % et le plus préférablement de 10 % à 40 % en poids de la composition totale.

6. Couche ou article de protection féminine selon l'une quelconque des revendications précédentes, où le dérivé d'ester ou un mélange de celui-ci est présent à un taux allant de 99,9 % à 0,1 %, de préférence de 97 % à 10 %, plus préférablement de 95 % à 40 % et le plus préférablement de 90 % à 60 % en poids de la composition totale.

7. Article de protection féminine selon l'une quelconque des revendications précédentes, choisi parmi un protège-slip et une serviette féminine.
